# EUROPÄISCHE PATENTSCHRIFT

(11) **EP 1 336 837 B1**
(45) Veröffentlichungstag und Bekanntmachung des Hinweises auf die Patenterteilung: **09.07.2008**
(21) Anmeldenummer: 03002954.0
(22) Anmeldetag: 11.02.2003
(51) Int. Cl.: G01N 25/52, G01N 33/22

(54) **Vorrichtung zur Bestimmung des Flammpunktes von Mineralölen**
Device for determining the flash point of mineral oils
Dispositif pour déterminer le point d'inflammation de l'huiles minérales

(30) Priorität: 14.02.2002 DE 10206021
(43) Veröffentlichungstag der Anmeldung: 20.08.2003
(73) Patentinhaber: Martechnic GmbH, 22459 Hamburg (DE)
(72) Erfinder: Herholdt, Ingo, 25421 Pinneberg (DE)
(74) Vertreter: Meyer, Ludgerus

(56) Entgegenhaltungen:
- DE-U- 20 005 845
- US-A- 2 823 824
- US-A- 2 911 821
- US-A- 3 293 904
- US-A- 5 182 940

## Beschreibung

Die Erfindung betrifft eine Vorrichtung zur Bestimmung des Flammpunktes von Mineralölen, insbesondere von Kraftstoffen, nach dem Penksy-Martens-Verfahren.

Nach dem Pensky-Martens-Verfahren wird der Flammpunkt von Mineralölen dadurch festgestellt, dass eine Probe des Mineralöls in einem topfförmigen Tiegel erwärmt wird und diejenige Temperatur ermittelt wird, bei der oberhalb des Mineralöls befindliche flüchtige Bestandteile der Probe mittels einer Zündquelle zur Entzündung bringbar sind. Ein solches Verfahren und eine zur Durchführung dieses Verfahrens geeignete Einrichtung sind aus der europäischen Norm EN 22719 bzw. der DIN 51758 bekannt. Ein solches Gerät besteht aus einem Tiegel, einem Verschlussdeckel und einer Heizkammer. An der Oberseite des Verschlussdeckels ist eine Abdeckscheibe drehbar befestigt, die um die Mittelachse des Deckels zwischen zwei Stoppern verdreht werden kann, so dass in der einen Endstellung der Abdeckscheibe mehrere Durchgänge des Verschlussdeckels vollständig geschlossen und in der anderen Endstellung die Durchgänge durch Öffnungen der Abdeckscheibe vollständig frei gegeben sind. In Ruhestellung der unter Federkraft stehenden Abdeckscheibe sind die Durchgänge des Verschlussdeckels verschlossen. Wenn die Abdeckscheibe aus der Ruhestellung gedreht wird, werden die Durchgänge des Verschlussdeckels geöffnet und die Flamme an der Spitze einer Zündquelle kann durch einen der Durchgänge in das Innere des Tiegels eintreten. Sofern sich oberhalb der in dem Tiegel befindlichen Probe zündfähige Bestandteile befinden, werden diese durch die Flamme entzündet.

Die Zündung der flüchtigen Bestandteile der Probe kann nur erfolgen, wenn die Probe eine bestimmte Temperatur erreicht hat. Der Flammpunkt des Mineralöls wird daher dadurch bestimmt, dass die niedrigste Temperatur festgestellt wird, die an einem in den Tiegel ragenden Thermometer ablesbar ist, bei der ein Entflammen stattfindet.

Aus der Gebrauchsmusterschrift DE 200 05 845 U1 ist eine Vorrichtung zur Bestimmung des Flammpunktes von Mineralölen bekannt, die einen Betätigungsmechanismus mit zwei ineinander greifenden Kegelrädern aufweist, von denen das erste Kegelrad starr mit der Abdeckscheibe verbunden ist, während das zweite Kegelrad starr mit einer schwenkbaren Zündquelle verbunden ist. Wird das erste Kegelrad betätigt, so wird die Abdeckscheibe verdreht, bis eine Öffnung der Abdeckscheibe einen Durchgang des Verschlussdeckels freigibt, während durch das zweite Kegelrad die Zündquelle verschwenkt wird, bis die Flamme an deren Spitze durch den Durchgang des Verschlussdeckels in den Innenraum des Tiegels eintritt und eventuell entzündbare Bestandteile der Probe entzündet.

Der Erfindung liegt die Aufgabe zu Grunde, eine verbesserte Vorrichtung zur Bestimmung der Flammpunktes von Mineralölen nach dem Pensky-Marten-Verfahren bereitzustellen.

Die Aufgabe der Erfindung wird durch die in Anspruch 1 angegebene Erfindung gelöst. Vorteilhafte Weiterbildungen der Erfindung sind in den Unteransprüchen angegeben.

Die Lösung der Aufgabe geht aus von einer Vorrichtung zur Bestimmung des Flammpunktes von Mineralölen nach dem Pensky-Martens-Verfahren, bei dem eine Probe des Mineralöls in einem topfförmigen Tiegel erwärmt wird und diejenige Temperatur ermittelt wird, bei der oberhalb des Mineralöls befindliche flüchtige Bestandteile der Probe mittels einer Zündquelle zur Entzündung bringbar sind. Der Tiegel weist einen aufsetzbaren Verschlussdeckel mit einer drehbaren Abdeckscheibe auf, die in ihrer Ruhestellung einen Durchgang des Verschlussdeckels verschließt und in einer anderen Stellung durch eine Öffnung freigibt. Ein Betätigungsmechanismus ist mit der Abdeckscheibe und der an den Durchgang heranführbaren Zündquelle derart verbunden ist, dass nach dem Freigeben des Durchgangs durch weitere Betätigung des Betätigungsmechanismus die Zündquelle bis in das Innere des Tiegels eingeführt werden kann.

Die Lösung der Aufgabe ist dadurch gekennzeichnet, dass die Zündquelle in einer Führung axial verschiebbar gelagert ist, dass der Betätigungsmechanismus einen Bowdenzug aufweist, der die verschiebbare Zündquelle und die Abdeckscheibe kraftschlüssig miteinander verbindet, und dass durch Verschieben der Zündquelle die Abdeckscheibe über den Bowdenzug verdreht werden kann, bis die Öffnung der Abdeckscheibe den Durchgang des Verschlussdeckels freigibt und die Zündquelle an die Öffnung herangeführt ist.

Die erfindungsgemäße Vorrichtung ist aus einfach herzustellenden Bauteilen zusammengesetzt, so dass die Herstellungskosten gering sind. Durch die verschiebbare Lagerung der Zündquelle in einer Führung kann mit einfachen Mitteln die Hin- und Herbewegung der Zündquelle auf die drehbare Abdeckscheibe übertragen werden. Auch kann die erfindungsgemäße Vorrichtung zu Reinigungszwecken problemlos zerlegt und anschließend zusammengesetzt werden.

Vorzugsweise ist vorgesehen, dass die Zündquelle einen Gasbrenner aufweist.

In einer besonderen Ausführungsform ist vorgesehen, dass die Größe der Flamme der Zündquelle einstellbar ist, so dass die Größe der Flamme während einer Versuchreihe konstant gehalten werden kann. Somit wird die Reproduzierbarkeit der Messergebnisse verbessert.

In einer besonderen Ausführungsform ist vorgesehen, dass der Tiegel und ein äußere Behälter, in den der Tiegel unter Beibehaltung eines ringförmigen Luftspalts eingesetzt werden kann, jeweils einen Griff aufweisen, die die Handhabung des erhitzten äußeren Behälters und Tiegels erleichtern.

In einer weiteren Ausführungsform ist vorgesehen, dass die Zündquelle einen befüllbaren Gastank zur Versorgung des Gasbrenners aufweist, um die erfindungsgemäße Vorrichtung auch an Orten ohne Gasversorgungssystem einsetzen zu können.

In einer besonderen Ausführungsform ist vorgesehen, dass die Zündquelle eine Piezo-Zündeinrichtung aufweist, um eine schnelles und einfaches Entzünden der Flamme an der Spitze der Zündquelle zu ermöglichen.

Die Erfindung wird nachstehend anhand eines Aufführungsbeispiels erläutert. Es zeigen:
- Fig. 1: einen Schnitt durch die erfindungsgemäße Vorrichtung,
- Fig. 2: eine Draufsicht auf die erfindungsgemäße Vorrichtung mit der Zündquelle in Ruhestellung, und
- Fig.3: eine Draufsicht auf die erfindungsgemäße Vorrichtung mit der Zündquelle in Arbeitstellung.

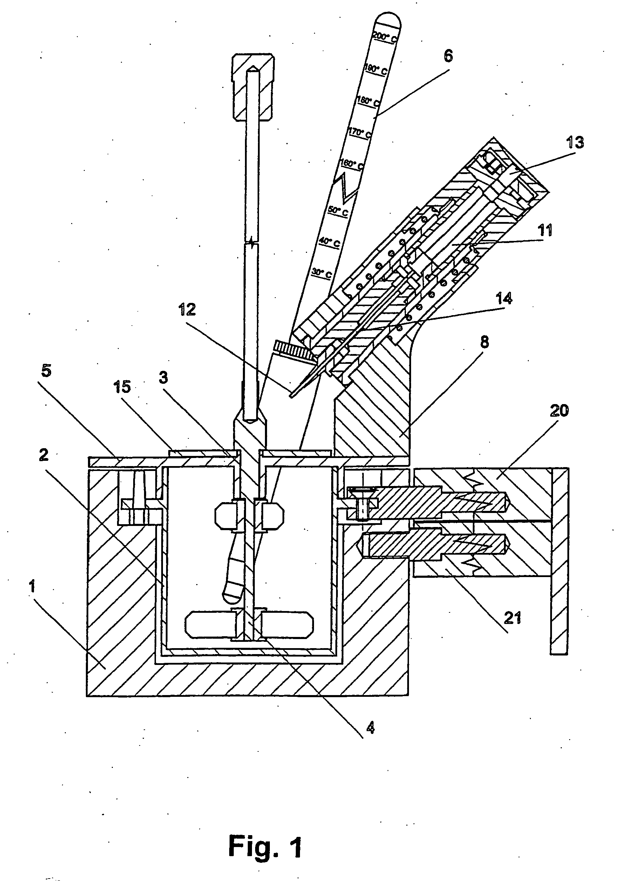

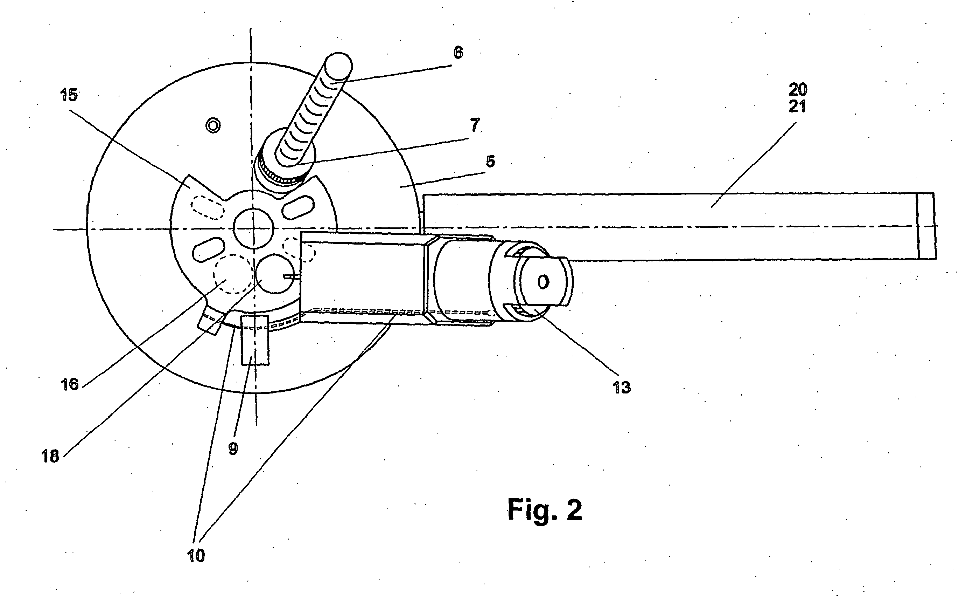

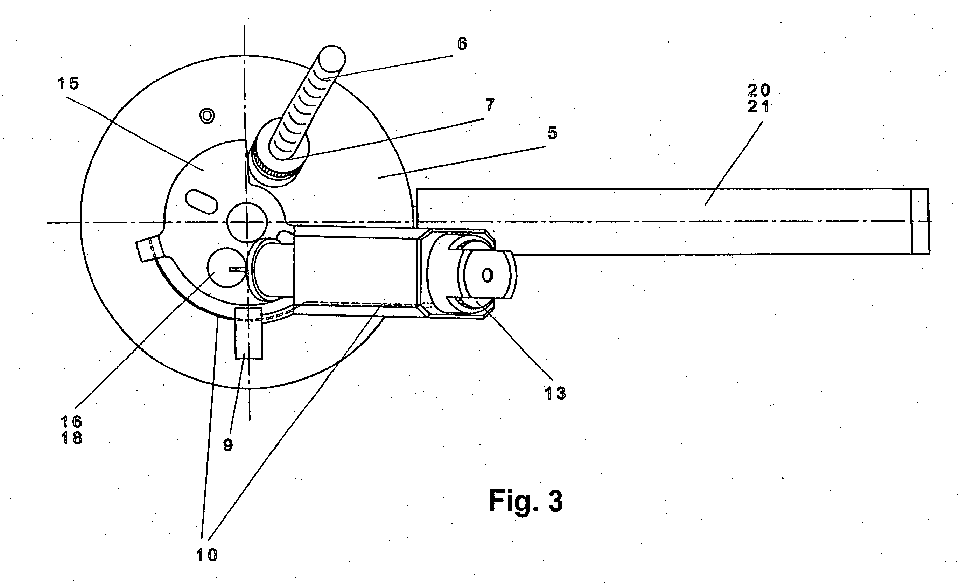

Es wird auf die Fig. 1 bis 3 Bezug genommen.

Die erfindungsgemäße Vorrichtung umfasst einen topfförmigen Tiegel 2, in den eine Probe des zu untersuchenden Mineralöls gegeben werden kann. Der Tiegel 2 wird in einen Außenbehälter 1 eingesetzt, wobei zwischen dem Außenbehälter 1 und dem Tiegel 2 ein Luftspalt verbleibt, um eine gleichmäßige und kontinuierliche Erwärmung der Probe zu gewährleisten.

Der Tiegel 2 ist mit einem Verschlussdeckel 5 verschlossen, der eine Zentralöffnung 3 aufweist, durch die die Antriebswelle eines Rührwerkes 4 geführt ist. Am Rand des Verschlussdeckels 5 ist eine Messöffnung 7 vorgesehen, durch die ein Thermometer 6 in den Innenraum des Tiegels 2 eingeführt ist. Desweiteren weist der Verschlussdeckel 5 einen Durchgang 16 auf.

Auf dem Verschlussdeckel 5 ist eine kreissektorförmige Abdeckscheibe 15 drehbar befestigt, die eine Öffnung 18 aufweist. Die Abdeckscheibe 15 ist in Drehrichtung federnd vorgespannt, so dass die Abdeckscheibe 15 in ihrer Ruheposition den Durchgang 16 abdeckt. Die Abdeckscheibe 15 kann so gedreht werden, dass die Öffnung 18 der Abdeckscheibe 15 mit dem Durchgang 16 des Verschlussdeckels 5 fluchtet und einen Durchgang in das Innere des Tiegels 2 bildet. Die Abdeckscheibe weist einen kreissektorförmigen Ausschnitt auf, der den Bereich des Verschlussdeckels freilässt, in dem die Messöffnung 7 mit dem eingesetzten Thermometer 6 angeordnet ist, so dass das eingesetzte Thermometer 6 die Drehbewegung der Abdeckscheibe nicht behindert.

An dem Verschlussdeckel 5 ist eine Führung 8 befestigt, in der eine Zündquelle 11 mittels eines Schlittens verschiebbar gelagert ist. Die Spitze 12 der Zündquelle 11 kann bis in den Innenraum des Tiegels 2 eintreten, wenn der Durchgang 16 des Verschlussdeckels 5 und die Öffnung 18 der Abdeckscheibe 15 übereinanderliegen. Der Schlitten mit der Zündquelle 11 und die Abdeckscheibe 15 sind durch einen Bowdenzug miteinander verbunden, der in einem Führungsblock 9 auf dem Verschlussdeckel 5 verankert ist. Ein Verschieben der Zündquelle 11 in Richtung auf dem Durchgang 16 lässt über den Bowdenzug die Abdeckscheibe 15 gleichzeitig verschwenken, bis der Durchgang 16 des Verschlussdeckels 5 und die Öffnung 18 der Abdeckscheibe 15 übereinanderliegen, so dass die Spitze 12 der Zündquelle 11 in den Durchgang des Tiegels 2 eintreten und in Abhängigkeit von der Temperatur der Gase im Tiegel 2 entzünden kann.

Die Zündquelle 11 besteht aus einem zweigeteilten, zylindrischen Grundkörper, der in die Führung 8 eingesetzt werden kann. Entlang der Längsachse des zylindrischen Grundkörpers durchläuft eine Gasleitung 14 die Zündquelle 11, so dass sich vorne an der Spitze 12 eine Austrittöffnung befindet, während an der Rückseite eine Stellschraube 13 zur Einstellung der Zündquelle vorgesehen ist.

Zur Untersuchung eines Mineralöls wird eine Probe in den Tiegel 2 gegeben und dieser mit dem Verschlussdeckel 5 verschlossen. Der verschlossene Tiegel 2 wird dann in den äußeren Behälter 1 eingesetzt, der mittels einer Wärmeplatte kontinuierlich erwärmt wird. Um die Probe gleichmäßig zu erwärmen, wird sie durch das Rührwerk 4 permanent umgewälzt.

Wird anhand des Thermometers 6 festgestellt, dass eine bestimmte untere Temperatur erreicht wurde, ab der mit der Bildung zündfähiger flüchtiger Bestandteile zu rechnen ist, wird die Zündquelle 11 gezündet und die Größe der Flamme an der Spitze 12 der Zündquelle 11 durch Regulierung des Gasflusses eingestellt. Nun erfolgt die erste Messung, bei der die Zündquelle 11 in der Führung 8 in Richtung auf die Abdeckscheibe 15 geschoben wird. Dabei wird durch den Bowdenzug 10 gleichzeitig die Abdeckscheibe 15 verdreht, bis der Durchgang 16 des Verschlussdeckels 5 von der Öffnung 18 der Abdeckscheibe 15 freigegeben ist, so dass durch weiteres Verschieben der Zündquelle 11 die Spitze 12 mit der Flamme in den Durchgang 16 und die Öffnung 18 eintreten kann und dort eventuell vorhandene zündfähige Bestandteile entzündet.

Falls sich bei dieser Temperatur nach ca. einer Sekunde keine zündfähigen Bestandteile in dem Tiegel 2 befinden, wird die Zündquelle 11 wieder zurückgezogen, so dass die Flamme wieder aus dem Tiegel 2 hervortritt und durch den Bowdenzug 10 die Abdeckscheibe 15 in ihre Ruhestellung verschwenkt wird, in der sie den Durchgang 16 verdeckt. Anschließend wird die Probe in dem Tiegel 2 weiter erwärmt und nach dem Erreichen einer höheren Temperatur die Messung wiederholt. Dieser Vorgang wird so lange wiederholt, bis sich über der Probe zündfähige Bestandteile gebildet haben, die mit der Zündquelle 11 zu Entzündung gebracht werden können, wobei zwischen den einzelnen Messungen die Größe der Flamme kontrolliert und durch Einstellen des Gasflusses korrigiert werden kann.

### Bezugszeichen

- 1: Behälter
- 2: Tiegel
- 3: Zentralöffnung
- 4: Rührwerk
- 5: Verschlussdeckel
- 6: Thermometer
- 7: Messöffnung
- 8: Führung
- 9: Führungsblock
- 10: Bowdenzug
- 11: Zündquelle
- 12: Spitze
- 13: Stellschraube
- 14: Gasleitung
- 15: Abdeckscheibe
- 16: Durchgang
- 18: Öffnung
- 20: Griff
- 21: Griff

## Patentansprüche

1. Vorrichtung zur Bestimmung des Flammpunktes von Mineralölen, insbesondere Kraftstoffen, nach dem Pensky-Martens-Verfahren, bei dem eine Probe des Mineralöls in einem topfförmigen Tiegel (2) erwärmt wird und diejenige Temperatur ermittelt wird, bei der oberhalb des Mineralöls befindliche flüchtige Bestandteile der Probe mittels einer Zündquelle (11) zur Entzündung bringbar sind, wobei der Tiegel (2) einen aufsetzbaren Verschlussdeckel (5) mit einer drehbaren Abdeckscheibe (15) aufweist, die in ihrer Ruhestellung einen Durchgang (16) des Verschlussdeckels (5) verschließt und in einer anderen Stellung durch eine Öffnung (18) freigibt, wobei ein Betätigungsmechanismus mit der Abdeckscheibe (15) und der an den Durchgang (16) heranführbaren Zündquelle (11) derart verbunden ist, dass nach dem Freigeben des Durchgangs (16) durch weitere Betätigung des Betätigungsmechanismus die Zündquelle (11) bis in das Innere des Tiegels (2) eingeführt werden kann,
**dadurch gekennzeichnet, dass**
die Zündquelle (11) in einer Führung (8) axial verschiebbar gelagert ist, dass der Betätigungsmechanismus einen Bowdenzug (10) aufweist, der die verschiebbare Zündquelle (11) und die Abdeckscheibe (15) kraftschlüssig miteinander verbindet, und dass durch Verschieben der Zündquelle (11) die Abdeckscheibe (15) über den Bowdenzug (10) verdreht werden kann, bis die Öffnung (18) der Abdeckscheibe (15) den Durchgang (16) des Verschlussdeckels (5) freigibt und die Zündquelle (11) an die Öffnung (18) herangeführt ist.

2. Vorrichtung nach Anspruch 1, **dadurch gekennzeichnet, dass** die Zündquelle (11) einen Gasbrenner aufweist.

3. Vorrichtung nach Anspruch 1 oder 2, **dadurch gekennzeichnet, dass** die Größe der Flamme der Zündquelle (11) einstellbar ist.

4. Vorrichtung nach einem der vorherigen Ansprüche, **dadurch gekennzeichnet, dass** der Tiegel (2) und ein äußere Behälter (1), in den der Tiegel (2) unter Beibehaltung eines ringförmigen Luftspalts eingesetzt werden kann, jeweils einen Griff (20, 21) aufweisen.

5. Vorrichtung nach Anspruch 2, **dadurch gekennzeichnet, dass** die Zündquelle (11) einen befüllbaren Gastank zur Versorgung des Gasbrenners aufweist.

6. Vorrichtung nach einem der vorherigen Ansprüche, **dadurch gekennzeichnet, dass** die Zündquelle (11) eine Piezo-Zündeinrichtung aufweist.

## Claims

1. Device for determining the flashpoint of mineral oils, particularly fuels, according to the Pensky-Martens method, whereby a sample of mineral oil is heated in a closed cup (2) and that temperature ascertained at which volatile ingredients of the sample located above the mineral oil can be brought to ignition by means of an ignition source (11), the cup (2) displaying a cover lid (5) that can be placed on it with a rotatable cover disc (15), which in its rest position closes a passage (16) in the cover lid (5) and in another position unblocks it via an aperture (18), an actuating mechanism being connected to the cover disc (15) and to the ignition source (11) that can be advanced to the passage (16), such that after the passage (16) is unblocked, the ignition source (11) can be introduced right into the inside of the cup (2) by further actuating the actuating mechanism,
**characterised in that**
the ignition source (11) is mounted to be axially translatable in a guide (8), that the actuating mechanism displays a Bowden wire (10) which connects the translatable ignition source (11) to the cover disc (15) in a non-positive locking manner and that the cover disc (15) can be twisted via the Bowden wire (10) by translating the ignition source (11), until the aperture (18) in the cover disc (15) unblocks the passage (16) in the cover lid (5) and the ignition source (11) is advanced to the aperture (18).

2. Device according to claim 1, **characterised in that** the ignition source (11) displays a gas burner.

3. Device according to claim 1 or 2, **characterised in that** the size of the flame of the ignition source (11) can be adjusted.

4. Device according to any of the aforementioned claims, **characterised in that** the cup (2) and an outer container (1), into which the cup (2) can be inserted while retaining a circular air gap, each display a handle (20,21).

5. Device according to claim 2, **characterised in that** the ignition source (11) displays a fillable gas tank for supplying the gas burner.

6. Device according to any of the aforementioned claims, **characterised in that** the ignition source (11) displays a Piezo ignition facility.

## Revendications

1. Dispositif pour définir le point d'inflammation d'huiles minérales, en particulier de carburants, selon le procédé Pensky-Martens, c'est-à-dire qu'on chauffe un échantillon de l'huile minérale dans un creuset en forme de godet (2) et on détermine la température à laquelle des composants volatils de l'échantillon qui se trouvent au-dessus de l'huile minérale peuvent s'enflammer à l'aide d'une source d'allumage (11), le creuset (2) comportant un couvercle apte à être posé (5) avec une plaque de recouvrement pivotante (15) qui ferme un passage (16) du couvercle (5), en position fermée, et dégage ce passage (16) grâce à une ouverture (18), dans une autre position, un mécanisme d'actionnement étant relié à la plaque de recouvrement (15) et à la source d'allumage (11) apte à être rapprochée du passage (16), de telle sorte qu'après le dégagement du passage (16), la poursuite de l'actionnement dudit mécanisme permet d'introduire la source d'allumage (11) à l'intérieur du creuset (2),
**caractérisé en ce que** la source d'allumage (11) est montée pour pouvoir coulisser axialement dans un guide (8), **en ce que** le mécanisme d'actionnement comporte une commande Bowden (10) qui relie par force la source d'allumage coulissante (11) et la plaque de recouvrement (15), et **en ce que** grâce au coulissement de la source d'allumage (11), la plaque de recouvrement (15) peut pivoter par l'intermédiaire de la commande Bowden (10) jusqu'à ce que l'ouverture (18) de la plaque de recouvrement (15) dégage le passage (16) du couvercle (5) et que la source d'allumage (11) soit rapprochée de l'ouverture (18).

2. Dispositif selon la revendication 1, **caractérisé en ce que** la source d'allumage (11) comporte un brûleur à gaz.

3. Dispositif selon la revendication 1 ou 2, **caractérisé en ce que** la taille de la flamme de la source d'allumage (11) est réglable.

4. Dispositif selon l'une des revendications précédentes, **caractérisé en ce que** le creuset (2) et un récipient extérieur (1) dans lequel on peut placer le creuset (2) en conservant un interstice annulaire ont chacun une poignée (20, 21).

5. Dispositif selon la revendication 2, **caractérisé en ce que** la source d'allumage (11) comporte un réservoir de gaz apte à être rempli, pour alimenter le brûleur à gaz.

6. Dispositif selon l'une des revendications précédentes, **caractérisé en ce que** la source d'allumage (11) comporte un dispositif d'allumage piézoélectrique.
